# EUROPÄISCHE PATENTANMELDUNG

(11) **EP 1 074 299 A2**
(43) Veröffentlichungstag der Anmeldung: **07.02.2001**
(21) Anmeldenummer: 00115123.2
(22) Anmeldetag: 12.07.2000
(51) Int. Cl.: B01J 23/62, B01J 23/89, B01J 21/06, C07C 5/333

(54) **Multikomponenten-Dehydrierungskatalysatoren**

(30) Priorität: 06.08.1999 DE 19937106
(71) Anmelder: BASF AKTIENGESELLSCHAFT, 67056 Ludwigshafen (DE)
(72) Erfinder: Heineke, Daniel, Dr., 67487 Maikammer (DE); Harth, Klaus, Dr., 67317 Altleiningen (DE); Stabel, Uwe, Dr., 67166 Otterstadt (DE)

(57) **Zusammenfassung**

Multikomponenten-Katalysatoren, die
a) Platin und Zinn und
b) Gallium, Indium, Cobalt und/oder Germanium,
c) gegebenenfalls Scandium, Yttrium und/oder Lanthan und
d) gegebenenfalls Alkali- und/oder Erdalkalimetalle
auf einem Zirkonoxid-Träger, der gegebenenfalls SiO₂, Al₂O₃ und/oder TiO₂ enthält, enthalten.

## Beschreibung

Die vorliegende Erfindung betrifft Multikomponenten-Katalysatoren, die a) Platin und Zinn, b) Gallium, Indium, Cobalt und/oder Germanium, c) gegebenenfalls Scandium, Yttrium und/oder Lanthan und d) gegebenenfalls Alkali- und/oder Erdalkalimetalle auf einem Zirkonoxid-Träger, der gegebenenfalls SiO₂, Al₂O₃ und/oder TiO₂ enthält, enthalten.

Aus der US-A-5,220,091 sind Katalysatoren bestehend aus Pt/Sn als Aktivkomponente auf einem Zn-Spinell-Träger zur Dehydrierung von kleinen Kohlenwasserstoffmolekülen wie Isobutan mit Wasserdampf als Verdünnungsmittel bekannt. Hinsichtlich ihrer Performance sind diese Katalysatoren verbesserungsbedürftig, denn es werden trotz hoher Verdünnung des Feed mit Wasserdampf (Verhältnis 4:1) bei hohen Reaktionstemperaturen von 600°C nur relativ geringe Umsätze und Selektivitäten erzielt. Ebenfalls verbesserungswürdig ist die Standzeit der Katalysatoren, denn es muß nach einer Betriebszeit von nur 7 h regeneriert werden.

Aus der US-A-4,788,371 sind Pt/Sn/Cs/Al₂O₃-Katalysatoren zur Dehydrierung von Kohlenwasserstoffen in Gegenwart von Wasserdampf (z.B. Wasserdampf/Propan 10:1) bekannt. Trotz des hohen Verdünnungsgrades werden nur geringe Umsätze von 21% erreicht.

Aus der WO-A-94/29021 sind Katalysatoren auf der Basis von Mischoxiden von Magnesium und Aluminium mit einem Edelmetall der Gruppe VIII, einem Metall der Gruppe IVa und gegebenenfalls einem Alkalimetall der Gruppe Ia des Periodensystems der Elemente zur Dehydrierung z.B. eines Gasgemisches aus H₂O/Propan/H₂/N₂ im Verhältnis 8:7:1:5 bekannt. Nachteilig für eine technische Anwendung dieser Katalysatoren ist ihre geringe Härte, die einen technischen Einsatz schwierig macht. Weiterhin sind diese Katalysatoren in ihrer Performance, insbesondere bei niedrigen Reaktionstemperaturen verbesserungsbedürftig. Ein weiterer Nachteil ist die aufwendige Fahrweise, die zur Erhaltung der Performance den Zusatz von Wasserstoff zum Feed und die Zumischung von Stickstoff zur weiteren Verdünnung erfordert.

Der vorliegenden Erfindung lag deshalb die Aufgabe zugrunde, den zuvor genannten Nachteilen abzuhelfen.

Demgemäß wurden neue und verbesserte Multikomponenten-Katalysatoren gefunden, die
a) Platin und Zinn,
b) Gallium, Indium, Cobalt und/oder Germanium,
c) gegebenenfalls Scandium, Yttrium und/oder Lanthan und
d) gegebenenfalls Alkali- und/oder Erdalkalimetalle
auf einem Zirkonoxid-Träger, der gegebenenfalls SiO₂, Al₂O₃ und/oder TiO₂ enthält, enthalten.

Zur Herstellung der erfindungsgemäßen Katalysatoren können Precursoren der Oxide des Zirkons, die sich durch Calcinieren in die Oxide umwandeln lassen, eingesetzt werden. Diese können nach bekannten Verfahren, zum Beispiel nach dem Sol-Gel-Verfahren, Fällung der Salze, Entwässern der entsprechenden Säuren, Trockenmischen, Aufschlämmen oder Sprühtrockrien hergestellt werden. Zum Beispiel kann zur Herstellung eines ZrO₂-Trägers zunächst ein wasserreiches Zirkonoxid der allgemeinen Formel ZrO₂ · xH₂O durch Fällung eines geeigneten Zirkon-haltigen Precursors hergestellt werden. Geeignete Precursoren des Zirkons sind zum Beispiel Zr(NO₃)₄, ZrOCl₂, oder ZrCl₄. Die Fällung selbst erfolgt durch Zugabe einer Base wie zum Beispiel NaOH, KOH, Na₂CO₃ oder NH₃ und ist beispielsweise in der EP-A-849 224 beschrieben.

Zur gezielten Herstellung von ZrO₂ mit speziellen Porenradienverteilungen können dem ZrO₂ verschiedene Polymere zugegeben werden, die durch Calcinierung teilweise oder vollständig entfernt werden, wobei Poren in definierten Porenradienbereichen entstehen. Die Mischung der beiden Komponenten kann beispielsweise durch einfaches mechanisches Vermischen oder durch Sprühtrocknen in einem Sprühturm erfolgen.

Besonders bewährt zur Herstellung von ZrO₂ mit bimodaler Porenradienverteilung hat sich die Verwendung von Polyvinylpyrrolidon (PVP). Wird dieses in einem Herstellschritt zum ZrO₂-Precursor gegeben, so entstehen nach dem Calcinieren Makroporen im Bereich von 200 bis 5000 nm. Ein weiterer Vorteil der Verwendung von PVP ist die leichtere Verformbarkeit des Trägers. So können aus frisch gefälltem wasserhaltigem ZrO₂ · xH₂O, das vorher bei 120°C getrocknet wurde, unter Zusatz von Polyvinylpyrrolidon und Ameisensäure mühelos Stränge mit guten mechanischen Eigenschaften hergestellt werden.

Außer reinem ZrO₂ können auch Mischoxide des Zirkons als Träger eingesetzt werden. Diese Mischoxide können als weitere Komponenten Oxide des Titans, Siliciums oder Aluminiums aufweisen. Besonders bewährt hat sich die Verwendung von ZrO₂ · xSiO₂-Mischoxiden mit Anteilen von 0,5 bis 10 Gew.-% SiO₂ bezogen auf ZrO₂ und die Verwendung von ZrO₂ · xAl₂O₃ · xSiO₂-Mischoxiden mit Anteilen von 0,5 bis 10 Gew.-% SiO₂ und Anteilen von 0,5 bis 60 Gew.-% Al₂O₃.

Die Mischoxide können z.B. durch gemeinsames Fällen, Trockenmischen der Precursoren oder gemeinsames Versprühen hergestellt werden. Besonders bewährt hat sich bei der Herstellung von ZrO₂ · xSiO₂-Mischoxiden das gemeinsame Versprühen einer Zirkonverbindung der allgemeinen Zusammensetzung ZrO₂ · xH₂O mit einem SiO₂-haltigen Sol wie Ludox™. Nach Trocknen dieser Mischung kann das Al₂O₃ z.B. in Form eines Precursors wie AlOOH (Böhmit) zugegeben werden. Nach gemeinsamem Verkneten kann daraus nach Calcinierung ein Mischoxid-Träger erhalten werden.

Die Träger der erfindungsgemäßen Katalysatoren weisen nach der Calcinierung im allgemeinen hohe BET-Oberflächen auf. Die BET-Oberflächen liegen im allgemeinen zwischen 40 und 250 m²/g, bevorzugt zwischen 50 und 200 m²/g, besonders bevorzugt zwischen 70 und 150 m²/g. Das Porenvolumen der erfindungsgemäßen Katalysatoren beträgt üblicherweise 0,2 bis 0,8 ml/g, bevorzugt 0,25 bis 0,5 ml/g. Der durch Hg-Porosimetrie bestimmbare mittlere Porendurchmesser der erfindungsgemäßen Katalysatoren liegt zwischen 3 und 20 nm, bevorzugt zwischen 4 und 15 nm.

Die Calcinierung der Träger kann zweckmäßigerweise nach dem Aufbringen der Aktivkomponenten erfolgen und wird bei Temperaturen von 400 bis 700°C, bevorzugt von 500 bis 650°C, besonders bevorzugt bei 560 bis 620°C durchgeführt. Die Calciniertemperatur sollte dabei üblicherweise mindestens so hoch sein wie die Reaktionstemperatur der Dehydrierung für die die erfindungsgemäßen Katalysatoren eingesetzt werden.

Die Dotierung der Träger mit einer basischen Verbindung kann entweder während der Herstellung, zum Beispiel durch gemeinsame Fällung oder nachträglich zum Beispiel durch Tränken des Mischoxides mit einer Alkali- oder Erdalkalimetallverbindung oder einer Verbindung der 3. Nebengruppe oder einer Seltenerdmetall-Verbindung erfolgen. Besonders geeignet zur Dotierung sind Kalium, Cäsium und Lanthan.

Die Aufbringung der dehydrieraktiven Komponenten Platin sowie erfindungsgemäß mindestens eines Elementes aus der Reihe Gallium, Indium, Germanium und/oder Cobalt, erfolgt in der Regel durch Tränkung mit geeigneten Metallsalzprecursoren. Statt durch Tränkung können die dehydrieraktiven Komponenten aber auch durch andere Verfahren wie beispielsweise Aufsprühen der Metallsalzprecursoren erfolgen. Geeignete Metallsalzprecursoren sind z.B. die Nitrate, Acetate und Chloride der entsprechenden Metalle, möglich sind auch komplexe Anionen der verwendeten Metalle. Bevorzugt werden Platin als H₂PtCl₆ oder Pt(NO₃)₂ eingesetzt. Geeignete Metallsalzprecursoren der bimetallischen Komponenten Gallium, Indium, Germanium und Cobalt sind z.B. die Nitrate, Acetate und Chloride der entsprechenden Metalle, möglich sind auch komplexe Anionen der verwendeten Metalle.

Bevorzugt wird Gallium als Ga(NO₃)₃ · xH₂O, Indium als In(NO₃)₃ · xB₂O, Germanium als GeCl₄ und Co als Co(CH₃COO)₂ eingesetzt. Die Aufbringung der bimetallischen Komponente kann gemeinsam mit der Platin-Komponente in der selben Lösung, vor der Aufbringung der Platin-Komponente oder nach der Aufbringung der Platin-Komponente erfolgen. Vorteilhaft ist die gemeinsame Aufbringung in einem Lösungsmittel, in dem sich sowohl die Platin-Komponente als auch die bimetallische Komponente lösen. Als Lösungsmittel für die Metallsalzprecursoren eignen sich Wasser genauso wie organische Lösungsmittel. Besonders geeignet sind niedere Alkohole wie Methanol und Ethanol.

Geeignete Precursoren sind auch die entsprechenden Metallsole, die nach einem der bekannten Verfahren, zum Beispiel durch Reduktion eines Metallsalzes in Gegenwart eines Stabilisators wie PVP mit einem Reduktionsmittel hergestellt werden können. Die Herstelltechnik wird in der DE-A-195 00 366 ausführlich behandelt.

Der Katalysator kann im Reaktor fest angeordnet oder z.B. in Form eines Wirbelbettes verwendet werden und eine entsprechende Gestalt haben. Geeignet sind z.B. Formen wie Splitt, Tabletten, Monolithe, Kugeln, oder Extrudate (Stränge, Wagenräder, Sterne, Ringe).

Der Gehalt an Alkali-, Erdalkalimetall oder an einem Metall der dritten Nebengruppe oder einem seltenen Erdmetall oder Zink liegt im allgemeinen zwischen 0 und 20 Gew.-%, bevorzugt zwischen 0,1 und 15 Gew.-%, besonders bevorzugt zwischen und 0,3 und 10 Gew.-%. Als Alkali- und Erdalkalimetallprecursor verwendet man in der Regel Verbindungen, die sich durch Calcinieren in die entsprechenden Oxide umwandeln lassen. Geeignet sind zum Beispiel Hydroxide, Carbonate, Oxalate, Acetate oder gemischte Hydroxycarbonate der Alkali- und Erdalkalimetalle.

Wird der Träger zusätzlich oder ausschließlich mit einem Metall der dritten Nebengruppe dotiert, so sollte man auch in diesem Fall von Verbindungen ausgehen, die sich durch Calcinieren in die entsprechenden Oxide umwandeln lassen. Wird Lanthan verwendet, so sind beispielsweise Lanthan-Oxid-Carbonat, La(OH)₃, La₃(CO₃)₂, La(NO₃)₃ oder Lanthanverbindungen die organische Anionen enthalten, wie La-Acetat, La-Formiat, oder La-Oxalat geeignet.

Der Gehalt der erfindungsgemäßen Katalysatoren an Platin beträgt in der Regel 0,05 bis 5 Gew.-%, bevorzugt 0,1 bis 1 Gew.-%, besonders bevorzugt 0,2 bis 0,5 Gew.-%. Der Gehalt der erfindungsgemäßen Katalysatoren an einem oder mehreren der Elemente aus der Reihe Gallium, Indium, Germanium und Cobalt beträgt in der Regel 0,05 bis 5 Gew.-%, bevorzugt 0,1 bis 1 Gew.-%, besonders bevorzugt 0,2 bis 0,5 Gew.-%.

Die Propan-Dehydrierung wird in der Regel bei Reaktionstemperaturen von 300 bis 800°C, bevorzugt 450 bis 700°C und einem Druck von 0,1 bis 100 bar, bevorzugt 0,1 bis 40 bar mit einer GHSV (Gas Hourly Space Velocity) von 100 bis 10.000 h⁻¹, bevorzugt 500 bis 2.000 h⁻¹ durchgeführt. Neben dem zu dehydrierenden Kohlenwasserstoff können im Feed Verdünnungsmittel wie beispielsweise CO₂, N₂, Edelgase oder Dampf zugegen sein.

Ein spezielles Merkmal der erfindungsgemäßen Katalysatoren ist, daß sie in Gegenwart von Wasserdampf aktiv sind und die damit verbundenen Vorteile wie Aufhebung der Gleichgewichtslimitierung, Verringerung der Verkokung und Verlängerung der Standzeiten genutzt werden können.

Gegebenenfalls kann zum Kohlenwasserstoff-Feed Wasserstoff zugegeben werden, wobei das Verhältnis von Wasserstoff zu Kohlenwasserstoffstrom von 0,1 bis 100 bevorzugt von 1 bis 20 betragen kann. Ein weiteres vorteilhaftes Merkmal der erfindungsgemäßen Katalysatoren ist jedoch, daß sie auch ohne den Zusatz von Wasserstoff betrieben werden können.

Neben der kontinuierlichen Zugabe eines Gases, insbesondere von Dampf, welches die Verkokung während der Reaktion verhindert, gibt es die Möglichkeit, den Katalysator durch Überleiten von Wasserstoff oder Luft von Zeit zu Zeit zu regenerieren. Die Regenerierung selbst findet bei Temperaturen im Bereich 300 bis 900°C, bevorzugt 400 bis 800°C mit einem freien Oxidationsmittel, vorzugsweise mit Luft oder in reduktiver Atmosphäre vorzugsweise mit Wasserstoff statt. Die Regenerierung kann bei Unterdruck, Normaldruck oder Überdruck betrieben werden. Bevorzugt sind Drücke im Bereich 0,5 bis 100 bar.

Für die Dehydrierung mit den erfindungsgemäßen Katalysatoren eignen sich Kohlenwasserstoffe beispielsweise C₂- bis C₁₆-Kohlenwasserstoffe wie Ethan, n-Propan, n-Butan, iso-Butan, n-Pentan, iso-Pentan, n-Hexan, n-Heptan, n-Octan, n-Nonan, n-Decan, n-Undecan, n-Dodecan, n-Tridecan, n-Tetradecan, n-Pentadecan, n-Hexadecan, bevorzugt C₂- bis C₈-Kohlenwasserstoffe wie Ethan, n-Propan, n-Butan, iso-Butan, n-Pentan, iso-Pentan, n-Hexan, n-Heptan, n-Octan, besonders bevorzugt C₂- bis C₄-Kohlenwasserstoffe wie Ethan, n-Propan, n-Butan und iso-Butan, insbesondere Propan und iso-Butan.

Propylen ist ein gefragtes Produkt, insbesondere zur Synthese von Polypropylen oder zur Synthese von funktionalisierten Monomeren und deren Polymerisationsprodukten. Eine Alternative zur Herstellung von Propylen durch Steamcracking von leichtem Naphtha ist die Dehydrierung von Propan.

Isobuten ist ein wichtiges Produkt, insbesondere zur Herstellung von MTBE (Methyl-tert.-butyl-ether). Es wird vor allem in den USA als Kraftstoffadditiv zur Erhöhung der Oktanzahl verwendet. Isobuten läßt sich analog zu Propylen durch Dehydrierung von Isobutan herstellen.

### Beispiele

### Katalysatorherstellung

### Beispiel 1

4835,82 g ZrO₂ · xH₂O mit einem ZrO₂-Gehalt von 89,8 % wurden in 12 Liter Wasser aufgeschlämmt und mit einem ULTRA-TURRAX™ T 50 (Firma Ika) zerkleinert. Zu dieser Suspension wurde 330,84 g eines SiO₂-Sol (Ludox™) mit einem SiO₂-Gehalt von 47,6 % gegeben. Die Sprühmaische wurde bei einem Feststoff/Wasser-Verhältnis von 1 : 3,7 in einem NIRO-Atomizer mit einer Kopf-Temperatur von 350°C, einer Ausgangstemperatur von 105 bis 110°C, einem Sprühdruck von 5,2 bar bei 28000 U/min versprüht, so daß ein weißes Pulver entstand. Das Sprühpulver wurde 2 h bei 600°C calciniert. 1416,5 g des Sprühpulvers wurden mit 400 g Pural™ SCF (Böhmit mit einem Glühverlust von 25 %) unter Zugabe von 25 g konz. HNO₃ (65 %ig) gemischt und 2,5 h verknetet. Der Knetansatz wurde mittels einer Strangpresse zu 3 mm Vollsträngen bei 50 bar verarbeitet. Die Schnitthärte betrug 51 N/Strang (SA = 22 N).

88 g des zuvor zu 1,6 bis 2 mm gesplitteten Trägers wurden mit einer Lösung, die 1,2063 g SnCl₂ · 2 H₂O und 0,6748 g H₂PtCl₆ · 6 H₂O und 1,1157 g Co(CH₃COO)₄ · 4 H₂O in 524 ml Ethanol enthielt, getränkt. Die überstehende Lösung wurde i. Vak. entfernt und der Rückstand 15 h bei 100°C getrocknet und anschließend 3 h bei 560°C calciniert. Anschließend wurde mit einer Lösung von 0,6595 g CsNO₃ und 1,1633 g KNO₃ in 219 ml Wasser getränkt und der Katalysator 15 h bei 100°C getrocknet und anschließend 3 h bei 560°C calciniert.

Der Katalysator hatte eine BET-Oberfläche von 95 m²/g. Durch Quecksilber-Porosimetrie-Messungen wurde ein Porenvolumen von 0,37 ml/g ermittelt und eine Porenfläche von 91 m²/g und ein mittlerer Porenradius von 18,6 nm errechnet.

Die Zusammensetzung des Katalysators ist Tabelle 1 zu entnehmen.

### Beispiel 2

Es wurde analog Beispiel 1 verfahren mit dem Unterschied, daß bei der Tränkung des gesplitteten Trägers statt Co(CH₃COO)₂ · 4H₂O 1,62 g Ga(NO₃)₃ · 9H₂O eingesetzt wurden.

Der Katalysator hatte eine BET-Oberfläche von 93 m²/g. Durch Quecksilber-Porosimetrie wurde ein Porenvolumen von 0,36 ml/g ermittelt und eine Porenfläche von 95 m²/g und ein mittlerer Porenradius von 17,7 nm errechnet.

Die Zusammensetzung des Katalysators ist Tabelle 1 zu entnehmen.

### Beispiel 3

Es wurde analog Beispiel 1 verfahren mit dem Unterschied, daß bei der Tränkung des gesplitteten Trägers statt Co(CH₃COO)₂ · 4H₂O 0,797 g GeCl₄ eingesetzt wurden.

Der Katalysator hatte eine BET-Oberfläche von 94 m²/g. Durch Quecksilber-Porosimetrie wurde ein Porenvolumen von 0,36 ml/g ermittelt und eine Porenfläche von 94 m²/g und ein mittlerer Porenradius von 17,8 nm errechnet.

Die Zusammensetzung des Katalysators ist Tabelle 1 zu entnehmen.

### Beispiel 4

Es wurde analog Beispiel 1 verfahren mit dem Unterschied, daß bei der Tränkung des gesplitteten Trägers statt Co(CH₃COO)₂ · 4H₂O 0,919 g In(NO₃)₃ · 5H₂O eingesetzt wurden.

Der Katalysator hatte eine BET-Oberfläche von 94 m²/g. Durch Quecksilber-Porosimetrie wurde ein Porenvolumen von 0,36 ml/g ermittelt und eine Porenfläche von 95 m²/g und ein mittlerer Porenradius von 18,3 nm errechnet.

Die Zusammensetzung des Katalysators ist Tabelle 1 zu entnehmen.

### Vergleichsbeispiel

Es wurde ein Katalysator nach der Vorschrift in WO-A-94/29021, Beispiel 1 zum Vergleich präpariert (Pt/Sn/Cs/Mg(Al)O).

### Katalysatortest

20 ml des Katalysators wurden in einen Rohrreaktor mit einem Innendurchmesser von 22 mm eingebaut. Der Katalysator wurde bei einer Temperatur von 580°C 30 min mit Wasserstoff versetzt, danach einem Gemisch aus 80% Stickstoff und 20% Luft (Magerluft) ausgesetzt, anschließend 15 min mit reinem Stickstoff versetzt, 30 min mit Wasserstoff reduziert und mit 20 Nl/h Propan (99,5 %ig) und H₂O im Molverhältnis Propan/Wasserdampf von 1 : 1 beaufschlagt. Der Druck betrug 1,5 bar, die GHSV betrug 1000 h⁻¹. Die Reaktionsprodukte wurden gaschromatographisch erfaßt.

Die Ergebnisse mit den Katalysatoren der Beipiele 1 bis 4 und des Vergleichsbeispiels sind in Tabelle 1 aufgeführt.

**Tabelle 1**

| Performance der Katalysatoren der Beispiele 1 bis 4 und des Vergleichsbeispiels in der Propan-Dehydrierung* | | | | | | | | | | | | |
|---|---|---|---|---|---|---|---|---|---|---|---|---|
| | | | | | | | | | Umsatz [%] nach | | Selektivität [%] nach | |
| Beispiel Nr.: | Pt [%] | Sn [%] | Met [%] | K [%] | Cs [%] | ZrO₂ [%] | Al₂O₃ [%] | SiO₂ [%] | 1h | 17h | 1h | 17h |
| 1 | 0,3 | 0,6 | 0,3 Co | 0,5 | 0,5 | 75,5 | 19,5 | 2,7 | 44 | 35 | 80 | 90 |
| 2 | 0,3 | 0,6 | 0,3 Ga | 0,5 | 0,5 | 75,6 | 19,5 | 2,7 | 41 | 36 | 83 | 93 |
| 3 | 0,3 | 0,6 | 0,3 Ge | 0,5 | 0,5 | 75,5 | 19,5 | 2,7 | 41 | 34 | 82 | 84 |
| 4 | 0,3 | 0,6 | 0,3 In | 0,5 | 0,5 | 75,6 | 19,5 | 2,7 | 41 | 37 | 83 | 88 |
| Vergleich | 0,3 | 0,6 | --- | --- | 0,5 | --- | --- | --- | 33 | 29 | 92 | 95 |

| | | | | | | | | | | | | |
|---|---|---|---|---|---|---|---|---|---|---|---|---|
| *) Versuchsbedingungen: 20 ml Katalysator, Splittgröße 1,6 bis 2 mm; 580°C; Propan/H₂O 1 : 1 (mol/mol); 20 Nl/h Propan; GHSV = 1000 h⁻¹; 1,5 bar. | | | | | | | | | | | | |
| **) Vergleichskatalysator Pt/Sn/Cs/Mg(Al)O aus WO-A-94/29021 Beispiel 1. | | | | | | | | | | | | |

## Patentansprüche

1. Multikomponenten-Katalysatoren die
a) Platin und Zinn,
b) Gallium, Indium, Cobalt und/oder Germanium,
c) gegebenenfalls Scandium, Yttrium und/oder Lanthan und
d) gegebenenfalls Alkali- und/oder Erdalkalimetalle
auf einem Zirkonoxid-Träger, der gegebenenfalls SiO₂, Al₂O₃ und/oder TiO₂ enthält, enthalten.

2. Multikomponenten-Katalysatoren nach Anspruch 1, dadurch gekennzeichnet, daß die Katalysatoren
a) 0,05 bis 2 Gew.-% Platin und 0,05 bis 5 Gew.-% Zinn,
b) 0,05 bis 5 Gew.-% Gallium, Indium, Cobalt und/oder Germanium,
c) 0 bis 5 Gew.-% Scandium, Yttrium und/oder Lanthan und
d) 0 bis 10 Gew.-% Alkali- und/oder Erdalkalimetalle
bezogen auf den Träger enthalten.

3. Multikomponenten-Katalysatoren nach einem der Ansprüche 1 oder 2, dadurch gekennzeichnet, daß diese als c) 0 bis 5 Gew.-% Lanthan und als d) 0,1 bis 5 Gew.-% Kalium und/oder Cäsium enthalten.

4. Verwendung der Katalysatoren nach einem der Ansprüche 1 bis 3 zur Dehydrierung von C₂- bis C₁₆-Kohlenwasserstoffen.

5. Verfahren zur Dehydrierung von C₂- bis C₁₆-Kohlenwasserstoffen gegebenenfalls in Gegenwart von Wasserdampf und eines Katalysators nach einem der Ansprüche 1 bis 4.
